(19) Europäisches Patentamt / European Patent Office / Office européen des brevets

(11) **EP 4 223 394 A1**

(12) **EUROPEAN PATENT APPLICATION**
published in accordance with Art. 153(4) EPC

(43) Date of publication:
**09.08.2023 Bulletin 2023/32**

(21) Application number: **21875425.7**

(22) Date of filing: **24.09.2021**

(51) International Patent Classification (IPC):
**B01D 63/02** (2006.01)  **B01D 69/02** (2006.01)
**B01D 69/06** (2006.01)  **B01D 71/56** (2006.01)
**D01F 6/60** (2006.01)

(52) Cooperative Patent Classification (CPC):
**B01D 63/02; B01D 69/02; B01D 69/06; B01D 71/56; D01F 6/60;** Y02A 20/131

(86) International application number:
**PCT/JP2021/035114**

(87) International publication number:
**WO 2022/071123 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 JP 2020165097**

(71) Applicants:
• **Unitika Ltd.**
  **Osaka-shi, Osaka 541-8566 (JP)**
• **National University Corporation Kobe University**
  **Kobe-shi, Hyogo 657-8501 (JP)**

(72) Inventors:
• **NAKAMURA, Ryota**
  **Uji-shi, Kyoto 611-0021 (JP)**
• **INOUE, Kuniko**
  **Uji-shi, Kyoto 611-0021 (JP)**
• **UMAKOSHI, Kyohei**
  **Uji-shi, Kyoto 611-0021 (JP)**
• **ONO, Takahiro**
  **Uji-shi, Kyoto 611-0021 (JP)**
• **MATSUYAMA, Hideto**
  **Kobe-shi, Hyogo 657-8501 (JP)**

(74) Representative: **Müller-Boré & Partner**
**Patentanwälte PartG mbB**
**Friedenheimer Brücke 21**
**80639 München (DE)**

(54) **NANOFILTRATION MEMBRANE AND MANUFACTURING METHOD THEREFOR**

(57)     An object of the present invention is to provide a nanofiltration membrane having a molecular weight cut-off of 200 to 1,000 and a high amount pf permeate for methanol, and suitable for use as an organic solvent nanofiltration membrane. A nanofiltration membrane formed using a polyamide resin, the nanofiltration membrane having a molecular weight cut-off of 200 to 1,000 and a methanol permeability of 0.03 L/(m$^2$·bar·h) or more.

FIG. 1

EP 4 223 394 A1

**Description**

Technical Field

The present invention relates to a nanofiltration membrane having a molecular weight cut-off of 200 to 1,000 and a high flux of methanol permeate, and suitable for use as an organic solvent nanofiltration membrane, and to a method for producing the nanofiltration membrane. The present invention also relates to a nanofiltration method using the nanofiltration membrane.

Background Art

Nanofiltration membranes have conventionally been used in the field of water purification for removal of pesticides, odorous components, and hardness components, and in industrial fields for pre-treatment of RO water and ultrapure water production, desalination of soy sauce and dairy products, and purification of amino acids and lactic acid, for example. In recent years, in view of the social background that requires energy savings and a reduction in the generation of carbon dioxide, attempts have been made to convert a distillation separation process that consumes much energy to a membrane separation process, in the fields of chemical production, refining, and recycling. In these uses, various organic solvents are used, and the substances to be separated are often low-molecular-weight components. Thus, this process may also be designated as organic solvent nanofiltration (OSN), and the membrane used in this process as an organic solvent nanofiltration membrane (OSN membrane).

There are various definitions of nanofiltration, and the metes and bounds thereof are not fully clear; however, the IUPAC recommends that nanofiltration is defined as using a porous material with a pore size in the range of 2 nm or less. Since nanofiltration is intended for selection of substances with a size larger than in reverse osmosis, it may also be defined as using a porous material with a pore size in the range of 1 to 2 nm. On the other hand, pore sizes in nanofiltration are difficult to observe and measure even with an electron microscope, and additionally, these pore sizes vary. Therefore, a representative pore size of a membrane is not considered as sufficient to indicate the separation performance of the membrane, and the molecular weight cut-off is typically used as an index of the separation performance. The size of a target substance that can be separated by a nanofiltration membrane is not clearly defined around the molecular weight cut-off, and has a certain range. Specifically, in general, nanofiltration is classified as having a molecular weight cut-off of 200 to 1,000. As described below, the simple term "nanofiltration" or "nanofiltration membrane" refers to filtration in which the molecular weight cut-off is set in the range of 200 to 1,000 or a filtration membrane with a molecular weight cut-off in the range of 200 to 1,000.

Phase separation methods are widely used industrially to produce filtration membranes using polymer materials as raw materials. Such phase separation methods are roughly divided into non-solvent induced phase separation (NIPS) and thermally induced phase separation (TIPS). NIPS is a method in which a polymer material is dissolved in a good solvent to prepare a homogeneous polymer solution, and this polymer solution is immersed in a non-solvent, which induces phase separation by the entry of the non-solvent and the dissolution of the good solvent out into the external atmosphere. NIPS produces a finger-like structure with macrovoids; advantages of NIPS include: using a simple apparatus; allowing a dense layer to be easily formed on the surface, and allowing the flow rate to be easily increased; and having a long history and a track record for use in the production of many porous membranes. Disadvantages of NIPS include: the strength of the filtration membrane tends to be insufficient; and a good solvent that dissolves at room temperature is required. On the other hand, TIPS is a relatively new method; it is a method in which a solvent that dissolves a polymer material at a high temperature but not at a low temperature is selected as the solvent, and a homogeneous polymer solution obtained by dissolving the polymer in the solvent at a high temperature is cooled to a temperature less than or equal to the binodal line that is a boundary between the one phase region and the two phase region, which induces phase separation, and allows the structure to be fixed by crystallization or glass transition of the polymer. TIPS tends to produce a sponge-like homogeneous structure; typical advantages of TIPS include: providing a high strength; and applicable to polymers for which solvents that dissolve the polymers at low temperatures do not exist; while typical disadvantages include: using a complicated apparatus; and it is difficult to make a dense structure such as a nanofiltration membrane.

Organic solvent filtration membranes need to have resistance to a wide range of organic solvents. For example, polyamide filtration membranes formed using polyamide resins are known as filtration membranes with resistance to a wide range of organic solvents.

Regarding polyamide filtration membranes produced using NIPS, for example, it is known that an asymmetric polyamide hollow fiber composed of a thin separation membrane and a thick support membrane is obtained by extruding a spinning solution containing 15 to 25% by weight of polyamide and 5 to 20% by weight of polyethylene glycol, together with formic acid and a coagulating core liquid, into a precipitation solution with a difference between the pH values of the coagulating core and the precipitation solution of 3 or more, and then by stretching the hollow fiber in a wet state, followed by drying (see, for example, Patent Document 1).

One known polyamide filtration membrane produced using TIPS is an asymmetric hollow fiber membrane having, on an outer surface, a semipermeable layer with a thickness of 0.1 $\mu$m or more and 10 $\mu$m or less, wherein an outer diameter is 80 $\mu$m or more and 450 $\mu$m or less, and an inner diameter is 40 $\mu$m or more and 350 $\mu$m or less, and the hollow fiber membrane contains 70% by mass or more of at least one aliphatic polyamide selected from the group consisting of polyamide 4, polyamide 6, polyamide 11, polyamide 12, polyamide 46, polyamide 66, and polyamide 610 (see, for example, Patent Document 2). This hollow fiber membrane is described as having high water permeation performance or high moisture permeation performance and a high selectivity.

Another known polyamide filtration membrane produced using TIPS is an ultrafiltration membrane formed using a polyamide resin, wherein a dense layer is formed on at least one surface (see, for example, Patent Document 3). This ultrafiltration membrane is described as having excellent resistance to various types of organic solvents, and being capable of stably maintaining the membrane properties even when contacted with various types of organic solvents that are industrially used.

Prior Art Documents

Patent Documents

Patent Document 1: Japanese Patent Laid-Open Publication No. SHO58-65009
Patent Document 2: Japanese Patent Laid-Open Publication No. 2015-198999
Patent Document 3: Japanese Patent Laid-Open Publication No. 2016-193430

Summary of Invention

Technical Problem

When filtration is performed using a nanofiltration membrane, the pressure is increased from the viewpoint of ensuring a sufficient permeability of the liquid to be filtered (flux of permeate). The asymmetric hollow fiber membrane disclosed in Patent Document 1 is evaluated for flux of permeate under a pressure of 0.2 bar (= 0.02 MPa). However, research by the present inventors has shown that, because the asymmetric hollow fiber membrane disclosed in Patent Document 1 is produced using NIPS, the membrane of the asymmetric polyamide hollow fiber is broken when filtration is performed at a pressure of about 0.3 MPa, and thus, organic solvent nanofiltration at a molecular weight cut-off of 200 to 1,000 cannot be performed.

The hollow fiber membrane disclosed in Patent Document 2, which is produced using TIPS, has a strength to withstand organic solvent nanofiltration; however, research by the present inventors has shown that when organic solvent nanofiltration at a molecular weight cut-off of 200 to 1,000 is performed, the membrane is not observed to provide a sufficient flux of permeate, and thus, organic solvent nanofiltration cannot be efficiently performed.

The ultrafiltration membrane disclosed in Patent Document 3 has not been evaluated for performance in terms of organic solvent nanofiltration at a molecular weight cut-off of 200 to 1,000, and further research is required to perform organic solvent nanofiltration while ensuring a sufficient flux of permeate, using the technology disclosed in Patent Document 3.

It is therefore an object of the present invention to provide a nanofiltration membrane having a molecular weight cut-off of 200 to 1,000 and a high flux of permeate for methanol, and suitable for use as an organic solvent nanofiltration membrane.

Solution to Problem

As a result of extensive research to solve the above-described problem, the present inventors have found that a nanofiltration membrane having a molecular weight cut-off of 200 to 1,000 and a high flux of methanol permeate can be obtained by producing a polyamide filtration membrane by employing specific production conditions in TIPS.

Specifically, the inventors have found that when the below-described steps (1) to (3) are satisfied in the production of a polyamide filtration membrane, a nanofiltration membrane having a molecular weight cut-off of 200 to 1,000 and a methanol permeability of 0.03 L/(m$^2$·bar·h) or more can be obtained. The present invention has been completed by conducting further research based on this finding.

(1) Preparing a dope solution by dissolving a polyamide resin at a concentration of 25% by mass or more in an organic solvent at a temperature of 100°C or more, the organic solvent having a boiling point of 150°C or more and incompatible with the polyamide resin at a temperature of less than 100°C.

(2) Extruding the dope solution in a predetermined shape into a coagulation bath at 100°C or less to solidify the polyamide resin into a membrane, wherein at least one surface of the dope solution extruded in the predetermined shape is contacted with a coagulation liquid containing polyethylene glycol and/or polypropylene glycol with an average molecular weight of 400 to 1,000 to form a nanofiltration membrane.

(3) Removing the coagulation liquid from the nanofiltration membrane formed in (2) above.

In summary, the present invention provides embodiments of the invention as set forth below:

Item 1. A nanofiltration membrane formed using a polyamide resin, the nanofiltration membrane having a molecular weight cut-off of 200 to 1,000 and a methanol permeability of 0.03 L/(m²·bar·h) or more.

Item 2. The nanofiltration membrane according to item 1, which has a molecular weight cut-off of 250 to 990.

Item 3. The nanofiltration membrane according to item 1, which is a hollow fiber membrane with an outer diameter of 450 μm or more.

Item 4. The nanofiltration membrane according to any one of items 1 to 3, wherein the polyamide resin consists of only one aliphatic polyamide resin having methylene and amide groups at a molar ratio of $-CH_2-$ : $-NHCO- = 4:1$ to $10:1$.

Item 5. The nanofiltration membrane according to any one of items 1 to 4, wherein the polyamide resin is polyamide 6.

Item 6. The nanofiltration membrane according to any one of items 1 to 5, which is used for organic solvent nanofiltration.

Item 7. A nanofiltration method comprising subjecting a fluid to be treated containing a solute or particles to filtration treatment, using the nanofiltration membrane according to any one of items 1 to 6.

Item 8. The nanofiltration method according to item 7, wherein a solvent contained in the fluid to be treated is an organic solvent.

Item 9. A nanofiltration membrane module comprising the nanofiltration membrane according to any one of items 1 to 6, the nanofiltration membrane being housed in a module casing.

Item 10. A method for producing a nanofiltration membrane comprising the following first to third steps:

the first step of preparing a dope solution by dissolving a polyamide resin at a concentration of 25% by mass or more in an organic solvent at a temperature of 100°C or more, the organic solvent having a boiling point of 150°C or more and incompatible with the polyamide resin at a temperature of less than 100°C;

the second step of extruding the dope solution in a predetermined shape into a coagulation bath at 100°C or less to solidify the polyamide resin into a membrane, wherein at least one surface of the dope solution extruded in the predetermined shape is contacted with a coagulation liquid containing polyethylene glycol with an average molecular weight of 400 to 1,000 and/or polypropylene glycol with an average molecular weight of 400 to 1,000 to form a nanofiltration membrane; and

the third step of removing the coagulation liquid from the nanofiltration membrane formed in the second step.

Item 11. The method for producing a nanofiltration membrane according to item 10, which is a method for producing the nanofiltration membrane in the form of a hollow fiber membrane,

wherein the second step is the step of using a double-tube nozzle for hollow fiber production with a double-tube structure to discharge the dope solution from an outer annular nozzle while simultaneously discharging an internal coagulation liquid from an inner nozzle to immerse the dope solution and the internal coagulation liquid in a coagulation bath, and

a coagulation liquid containing polyethylene glycol with an average molecular weight of 400 to 1,000 and/or polypropylene glycol with an average molecular weight of 400 to 1,000 is used as at least one of the internal coagulation liquid and the coagulation bath.

Item 12. The method for producing a nanofiltration membrane according to item 10 or 11, which comprises the step of uniaxially stretching the nanofiltration membrane after the third step simultaneously with or after a drying treatment.

Effects of Invention

The nanofiltration membrane of the present invention is excellent in terms of flux of permeate, and can achieve improved productivity, energy savings, and lower costs in production processes in various fields of industries. Moreover, the nanofiltration membrane of the present invention has excellent resistance to many types of organic solvents, and can stably maintain the membrane properties even when it is contacted with various types of organic solvents that are industrially used, and thus, is suitable for use in organic solvent nanofiltration, and can provide a novel industrial process such as a substitute for distillation.

Furthermore, the nanofiltration membrane of the present invention is also applicable to a conventional aqueous filtration process, and, by virtue of its high hydrophilicity, the nanofiltration membrane of the present invention can have improved removal performance by an adsorption effect for a substance to be removed that is hydrophilic, while on the other hand, the nanofiltration membrane of the present invention exhibits less adsorption of hydrophobic substances, which can prevent fouling caused by the hydrophobic substances covering the membrane surface and reducing the treatment flow rate, thereby achieving an efficient filtration process.

Brief Description of Drawings

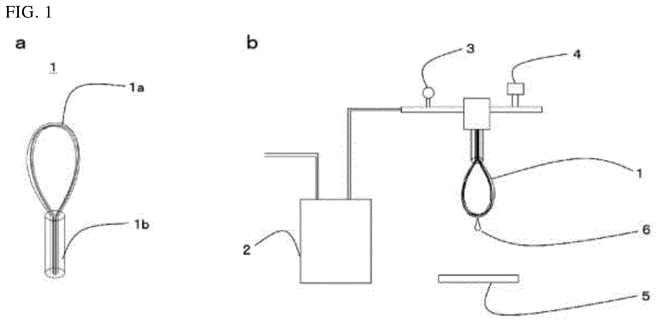

Fig. 1a is a schematic diagram of a module for use in measuring the methanol permeability; and Fig. 1b is a schematic diagram of the apparatus for use in measuring the methanol permeability.

Description of Embodiments

1. Definitions

As used herein, the term "nanofiltration membrane" refers to a filtration medium with a molecular weight cut-off of 200 to 1,000. The term "nanofiltration" refers to a filtration process performed using the nanofiltration membrane.

As used herein, the term "organic solvent nanofiltration membrane" refers to a nanofiltration membrane used in a filtration process for a fluid to be treated containing an organic solvent. The term "organic solvent nanofiltration" refers to a nanofiltration process performed for the fluid to be treated containing an organic solvent.

2. Nanofiltration Membrane

A nanofiltration membrane of the present invention is a nanofiltration membrane formed using a polyamide resin, the nanofiltration membrane having a molecular weight cut-off of 200 to 1,000 and a methanol permeability of 0.03 L/(m$^2$·bar·h) or more. The nanofiltration membrane of the present invention will be hereinafter described in detail.

[Constituent Material]

The nanofiltration membrane of the present invention is formed of a polyamide resin. When a polyamide resin is used as a constituent resin of the nanofiltration membrane of the present invention, the nanofiltration membrane can have resistance to a wide range of organic solvents.

While the type of polyamide resin to be used as the constituent resin is not limited, examples include polyamide homopolymers, polyamide copolymers, or mixtures thereof. Specific examples of polyamide homopolymers include polyamide 6, polyamide 66, polyamide 46, polyamide 610, polyamide 612, polyamide 11, polyamide 12, polyamide MXD6, polyamide 4T, polyamide 6T, polyamide 9T, and polyamide 10T. Specific examples of polyamide copolymers include copolymers of polyamide with polyethers such as polytetramethylene glycol and polyethylene glycol. While the proportion of the polyamide component in the polyamide copolymer is not limited, the content of the polyamide component is, for example, preferably 70 mol% or more, more preferably 80 mol% or more, still more preferably 90 mol% or more, and particularly preferably 95 mol% or more. When the proportion of the polyamide component in the polyamide copolymer satisfies the above-defined range, the nanofiltration membrane of the present invention can be provided with superior resistance to organic solvents.

From the viewpoint of further improving the resistance to a wide range of organic solvents, one preferred example of the polyamide resin to be used as the constituting resin is only one aliphatic polyamide resin having methylene and amide groups at a molar ratio of -CH$_2$- : -NHCO- = 4:1 to 10:1.

While the polyamide resin to be used as the constituent resin may or may not be crosslinked, the polyamide resin is preferably not crosslinked from the viewpoint of reducing production costs.

While the relative viscosity of the polyamide resin is not limited, it is, for example, 2.0 to 7.0, preferably 3.0 to 6.0, and more preferably 2.0 to 4.0. When the polyamide resin has this range of relative viscosities, the moldability and the ease of controlling phase separation during the production of the nanofiltration membrane are improved, and the nanofiltration membrane can be provided with excellent shape stability. As used herein, the term "relative viscosity" refers to the value measured with an Ubbelohde viscometer at 25°C, using a solution formed by dissolving 1 g of the polyamide resin in 100 mL of 96% sulfuric acid.

In the present invention, the polyamide resins to be used as constituent resins may be used alone or in combination.

In addition to the above-described polyamide resin, the nanofiltration membrane of the present invention may optionally contain a filler as long as it does not interfere with the effect of the present invention. The inclusion of a filler can improve the strength, elongation, and elastic modulus of the nanofiltration membrane. In particular, the inclusion of a filler also achieves the effect of making the nanofiltration membrane resistant to deformation even when a high pressure is applied during filtration. While the type of filler to be added is not limited, examples include fibrous fillers, such as glass fibers, carbon fibers, potassium titanate whiskers, zinc oxide whiskers, calcium carbonate whiskers, wollastonite whiskers, aluminum borate whiskers, aramid fibers, alumina fibers, silicon carbide fibers, ceramic fibers, asbestos fibers, gypsum fibers, and metal fibers; silicates, such as talc, hydrotalcite, wollastonite, zeolite, sericite, mica, kaolin, clay, pyrophyllite, bentonite, asbestos, and alumina silicate; metal compounds, such as silicon oxide, magnesium oxide, alumina, zirconium oxide, titanium oxide, and iron oxide; carbonates, such as calcium carbonate, magnesium carbonate, and dolomite; sulfates, such as calcium sulfate and barium sulfate; metal hydroxides, such as calcium hydroxide, magnesium hydroxide, and aluminum hydroxide; and inorganic materials, for example, non-fibrous fillers, such as glass beads, glass flakes, glass powder, ceramic beads, boron nitride, silicon carbide, carbon black, silica, and graphite. These fillers may be used alone or in combination. Preferred among these fillers are talc, hydrotalcite, silica, clay, and titanium oxide, and more preferred are talc and clay.

While the filler content is not limited, it is, for example, 5 to 100 parts by mass, preferably 10 to 75 parts by mass, and still more preferably 25 to 50 parts by mass, per 100 parts by mass of the polyamide resin. The inclusion of a filler at this content leads to an improvement in the strength, elongation, and elastic modulus of the nanofiltration membrane.

The nanofiltration membrane of the present invention may also optionally contain additives such as thickeners, antioxidants, surface modifiers, lubricants, and surfactants, in order to control the pore size or improve the membrane performance, for example.

[Shape and Structure]

While the shape of the nanofiltration membrane of the present invention is not limited and may be selected from any shapes such as a hollow fiber membrane and a flat sheet membrane, a hollow fiber membrane is preferred in the present invention because, it has a large filtration area per unit volume of the module, and enables efficient filtration treatment.

In the nanofiltration membrane of the present invention, a dense layer is formed on at least one surface thereof. As used herein, the term "dense layer" refers to a region where a collection of dense micropores is present, and substantially no pores are observed to be present in a scanning electron microscope (SEM) image at 10,000x magnification. In the nanofiltration membrane of the present invention, the dense layer portions are mostly responsible for the filtration performance such as methanol permeability and molecular weight cut-off. When the nanofiltration membrane is a flat sheet membrane, observation of the dense layer with a scanning electron microscope may be performed by cutting the membrane into an appropriate size, placing the sample on the sample stage, subjecting the sample to vapor deposition treatment with Pt, Au, Pd, or the like, and observing the sample. When the nanofiltration membrane is a hollow fiber membrane, observation of the dense layer present on the outer surface may be performed in the same manner as for a flat sheet membrane, by cutting the membrane into an appropriate size, placing the sample on the sample stage, subjecting the sample to vapor deposition treatment with Pt, Au, Pd, or the like, and observing the sample. On the other hand, observation of the dense layer present on the lumen-side surface may be performed by cutting the hollow fiber membrane in the longitudinal direction with a sharp knife such as a scalpel to expose the lumen-side surface, cutting the resulting sample into an appropriate size, placing the sample on the sample stage, subjecting the sample to vapor deposition treatment with Pt, Au, Pd, or the like, and observing the sample.

While the thickness of the dense layer in the nanofiltration membrane of the present invention is not limited, it is, for example, 10 to 2,000 nm, preferably 100 to 1,500 nm, more preferably 200 to 1,000 nm, still more preferably 400 to 1,000 nm, and particularly preferably 440 to 930 nm. As used herein, the thickness of the dense layer is the value determined by measuring, in a SEM image of a cross section of the nanofiltration membrane at 10,000x magnification, distances (thicknesses) of 10 or more regions where substantially no pores are observed to be present, and calculating the average value of the measurements.

It is only required that the dense layer be formed on at least one surface of the nanofiltration membrane of the present invention. For example, when the nanofiltration membrane of the present invention is a hollow fiber membrane, it is only required that the dense layer be formed on at least either one of the lumen-side surface and the outer surface. For example, when the nanofiltration membrane of the present invention is in the form of a flat sheet membrane, it is only required that the dense layer be formed on at least either one of the front surface and the back surface. From the viewpoint of satisfactorily imparting the below-described molecular weight cut-off and methanol permeability to the nanofiltration membrane, one preferred example of the nanofiltration membrane of the present invention is an embodiment in which the dense layer is provided on only one surface. One preferred example of the nanofiltration membrane of the present invention when it is a hollow fiber membrane is an embodiment in which the dense layer is provided on the lumen-side surface, but is not provided on the outer surface.

In the nanofiltration membrane of the present invention, regions other than the dense layer have a porous structure. The regions other than the dense layer may also be designated as "porous regions", hereinafter. The term "porous regions" specifically refers to regions where pores are observed to be substantially present in a scanning electron microscope (SEM) image at 2,000x magnification. Since the dense layer portions substantially determine the performance of the nanofiltration membrane of the present invention, the porous regions can be considered as a so-called support layer. The pore size in the porous regions is not limited as long as it does not significantly interfere with fluid permeation and the strength to support the dense layer.

When the nanofiltration membrane of the present invention is a hollow fiber membrane, the outer diameter of the membrane may be set appropriately according to the use, the thickness of the dense layer, the flux of permeate to be imparted, and the like. In view of the relationship with the membrane strength, the pressure loss in the fluid flowing through the hollow portions, the buckling pressure, and the effective membrane area when the membranes are packed into a module, the outer diameter of the hollow fiber membrane is 450 $\mu$m or more, preferably 450 to 4,000 $\mu$m, more preferably 500 to 3,500 $\mu$m, still more preferably 700 to 3,000 $\mu$m, and particularly preferably 700 to 2,000 $\mu$m. Moreover, when the nanofiltration membrane of the present invention is a hollow fiber membrane, other examples of the range of the outer diameter include from 500 to 2,000 $\mu$m and from 500 to 1,980 $\mu$m. Furthermore, when the nanofiltration membrane of the present invention is a hollow fiber membrane, the inner diameter of the membrane is not limited but is, for example, 100 to 3,000 $\mu$m, preferably 200 to 2,500 $\mu$m, more preferably 300 to 2,000 $\mu$m, and still more preferably 300 to 1,500 $\mu$m. Moreover, when the nanofiltration membrane of the present invention is a hollow fiber membrane, other examples of the range of the inner diameter include from 300 to 1,260 $\mu$m. As used herein, each of the outer and inner diameters of the hollow fiber membrane is the value determined by observing five hollow fiber membranes with an optical microscope at 200x magnification, measuring the outer and inner diameters (both at the point of the maximum diameter) of each hollow fiber membrane, and calculating the average value of each of the outer and inner diameters.

While the thickness of the nanofiltration membrane of the present invention may be set appropriately according to the use or shape of the nanofiltration membrane, the thickness of the dense layer, the flux of permeate to be imparted, and the like, it is, for example, 50 to 600 $\mu$m, and preferably 100 to 350 $\mu$m, from the viewpoint that nanofiltration is performed under high-pressure operation. Other examples of the range of the thickness of the nanofiltration membrane of the present invention include from 150 to 750 $\mu$m and from 200 to 720 $\mu$m. When the nanofiltration membrane of the present invention is in the form of a hollow fiber, the thickness of the nanofiltration membrane is the value calculated by dividing the outer diameter minus the inner diameter by 2.

[Molecular Weight Cut-Off and Methanol Permeability]

The nanofiltration membrane of the present invention has a molecular weight cut-off of 200 to 1,000 and a methanol permeability of 0.03 L/(m$^2$·bar·h) or more, and thus, can exhibit a high flux of permeate for an organic solvent while rejecting permeation of low-molecular-weight substances with molecular weights of 1,000 or less.

While the range of the molecular weight cut-off of the nanofiltration membrane of the present invention is not limited as long as it satisfies the range of 200 to 1,000, it is preferably 250 to 990, more preferably 250 to 950, still more preferably 500 to 900, and particularly preferably 600 to 850. Other examples of the range of the molecular weight cut-off of the nanofiltration membrane of the present invention include from 280 to 990. The molecular weight cut-off represents the pore size of the membrane that can reject 90% or more of a substance with a specific molecular weight, and is represented as the molecular weight of the substance that can be rejected.

As used herein, the molecular weight cut-off is the value determined using the following method. Using a 0.1% by mass solution of a polyethylene glycol of known molecular weight in pure water as the feed fluid, the feed fluid is filtered at a pressure of 0.3 MPa, and the fluid permeated through the membranes is collected. The concentration of the polyethylene glycol in the permeate is measured, and the rejection rate is calculated according to the equation shown below. Using polyethylene glycols of various molecular weights, the rejection rate for each polyethylene glycol is calculated, and, based on these results, a graph is plotted in which the horizontal axis represents the molecular weights of the polyethylene glycols used and the vertical axis represents the rejection rates for the polyethylene glycols, and the molecular weight at the intersection of the resulting approximate curve and the 90% rejection rate is determined as the molecular weight cut-off.

[Expression 1]

$$\text{Rejection rate (\%)} = \{(\text{concentration of the polyethylene glycol in the feed}$$

$$\text{fluid} - \text{concentration of the polyethylene glycol in the permeate})/\text{concentration of the}$$

$$\text{polyethylene glycol in the feed fluid}\} \times 100$$

While the methanol permeability of the nanofiltration membrane of the present invention is not limited as long as it is 0.03 L/(m²·bar·h) or more, it is usually 0.03 to 5.00 L/(m²·bar·h), preferably 0.10 to 3.00 L/(m²·bar·h), more preferably 0.30 to 1.50 L/(m²·bar·h), and still more preferably 0.30 to 1.20 L/(m²·bar·h). Other examples of the range of the methanol permeability of the nanofiltration membrane of the present invention include from 0.10 to 1.20 L/(m²·bar·h). When the nanofiltration membrane of the present invention has these ranges of methanol permeabilities, it provides excellent filtration efficiency, and allows filtration treatment to be performed at a rate that satisfies the practical level, in organic solvent nanofiltration.

When the nanofiltration membrane is a hollow fiber membrane, the methanol permeability as used herein is the value measured using internal pressure filtration, and is the value measured according to the following procedure: First, 10 hollow fiber membranes are cut into a length of 30 cm, aligned and bundled to prepare bundled hollow fiber membranes. Next, a rigid nylon tube with an outer diameter of 8 mm, an inner diameter of 6 mm, and a length of 50 mm is prepared, and, through one end opening of the tube, a rubber stopper with a length of about 20 mm is inserted to plug the one end opening. Next, a two-part mixture, room temperature curable epoxy resin is inserted into the opening opposite the opening with the rubber stopper to fill the inner space of the tube with the epoxy resin. Then, the bundled hollow fiber membranes prepared above are bent in a substantially U-shape, and both ends of the hollow fiber membranes are inserted into the tube filled with the epoxy resin, until the tips of the ends touch the rubber stopper. In this state, the epoxy resin is allowed to cure. Then, the rubber stopper-side region of the cured epoxy resin portions is cut together with the tube to produce a module in which the hollow portions at both ends of the hollow fiber membranes are open. Fig. 1a shows a schematic diagram of this module. Then, the module is mounted on the apparatus as shown in Fig. 1b, and methanol (100% methanol) at 25°C is passed through the inside of the hollow fiber membranes of the module at a pressure of about 0.3 MPa for a certain time. The volume of methanol permeated out of the hollow fiber membranes is determined, and the methanol permeability (L/(m²·bar·h)) is calculated according to the following equation:

[Expression 2]

$$\text{Methanol permeability} = \text{volume (L) of methanol permeated out of the hollow}$$

$$\text{fiber membranes}/[\text{inner diameter (m) of the hollow fiber membranes} \times 3.14 \times \text{effective}$$

$$\text{filtration length (m) of the hollow fiber membranes} \times 10 \text{ (number of membranes)} \times$$

$$\{[\text{pressure (bar)}\} \times \text{time (h)}]$$

Effective filtration length of the hollow fiber membranes: the length of the portions where the surface of the hollow fiber membranes in the module is not coated with the epoxy resin

When the nanofiltration membrane is a flat sheet membrane, the methanol permeability as used herein is the value measured using dead-end filtration, and is the value measured according to the following procedure: Using a flat sheet membrane cross-flow tester (for example, the Sepa-CF flat sheet membrane test cell from GE Water Technologies) connected to a high-pressure pump, the nanofiltration membrane in the form of a flat sheet membrane cut into a predetermined size (19.1 cm × 14.0 cm, effective membrane area in the cell: 155 cm²) is fixed to the cell, methanol at 25°C is passed, and methanol permeated at a predetermined pressure is collected. The volume (L) of the collected methanol is measured, and the methanol permeability (L/(m²·bar·h)) is determined according to the following equation:

[Expression 3]

$$\text{Methanol permeability} = \text{volume (L) of methanol permeated through the flat sheet membrane}/[\text{area (m}^2\text{) of the flat sheet membrane} \times \{[\text{pressure (bar)}\} \times \text{time (h)}]$$

In the nanofiltration membrane of the present invention, the molecular weight cut-off and the methanol permeability are not limited as long as they satisfy the above-defined ranges; however, from the viewpoint of satisfactorily achieving the removal performance for low-molecular-weight substances and permeability to organic solvents simultaneously; preferably, the molecular weight cut-off is 300 to 900, and the methanol permeability is 0.30 to 1.50 L/(m$^2$·bar·h); more preferably, the molecular weight cut-off is 500 to 900, and the methanol permeability is 0.30 to 1.50 L/(m$^2$·bar·h); still more preferably, the molecular weight cut-off is 500 to 850, and the methanol permeability is 0.40 to 0.95 L/(m$^2$·bar·h); and particularly preferably, the molecular weight cut-off is 600 to 850, and the methanol permeability is 0.40 to 0.95 L/(m$^2$·bar·h). Other examples of the range of the molecular weight cut-off and the range of the methanol permeability of the nanofiltration membrane of the present invention include the following: The molecular weight cut-off is 280 to 990, and the methanol permeability is 0.10 to 1.20 L/(m$^2$·bar·h); the molecular weight cut-off is 600 to 990, and the methanol permeability is 0.37 to 1.20 L/(m$^2$·bar·h); and the molecular weight cut-off is 600 to 880, and the methanol permeability is 0.41 to 0.92 L/(m$^2$·bar·h).

[Tensile Strength and Elongation]

The nanofiltration membrane of the present invention has a strength to withstand organic solvent nanofiltration, and specific properties thereof include, for example, having excellent tensile strength and elongation.

Specifically, the tensile strength of the nanofiltration membrane of the present invention when it is a hollow fiber membrane is, for example, 3 to 40 MPa, preferably 5 to 35 MPa, and more preferably 10 to 30 MPa. The elongation of the nanofiltration membrane of the present invention when it is a hollow fiber membrane is, for example, 50 to 400%, preferably 100 to 300%, and more preferably 100 to 250%.

As used herein, the tensile strength and elongation of the hollow fiber membrane each refer to the value measured according to the following procedure: The hollow fiber membrane is cut into a length of 100 mm, and subjected to a tensile test at a grip distance of 50 mm and a tensile speed of 50 mm/min, in an environment at a room temperature of 25°C and a humidity of 60%, to measure the load (N) and the elongation (mm) at break. Separately, the cross-sectional area (mm$^2$) of the hollow fiber membrane is determined. The tensile test and the measurement of the cross-sectional area are performed using five hollow fiber membranes, the average value of each of the load at break, the elongation at break, and the cross-sectional area is calculated, and the tensile strength and elongation are calculated using these average values, according to the following equations:

[Expression 4]

$$\text{Tensile strength (MPa)} = \text{load (N) at break/cross-sectional area (mm}^2\text{) of the hollow fiber membrane}$$

$$\text{Elongation (\%)} = (\text{elongation (mm) at break/grip distance (mm)}) \times 100$$

[Organic Solvent Resistance]

The nanofiltration membrane of the present invention has the property of stably maintaining the membrane structure by inhibiting a change in strength and elongation, even when it is contacted with various types of organic solvents (organic solvent resistance). More specifically, the nanofiltration membrane of the present invention has resistance to organic solvents such as alcohols, aprotic polar solvents, hydrocarbons, higher fatty acids, ketones, esters, and ethers. Specific examples of types of these organic solvents include the following:
Alcohols: primary alcohols, such as methanol, ethanol, n-propanol, n-butanol, and benzyl alcohol; secondary alcohols, such as isopropyl alcohol and isobutanol; tertiary alcohols, such as tertiary butyl alcohol; and polyhydric alcohols, such as ethylene glycol, diethylene glycol, triethylene glycol, tetraethylene glycol, propylene glycol, 1,3-butanediol, and glycerin.

Ketones: acetone, methyl ethyl ketone, cyclohexanone, diisopropyl ketone, and the like.

Ethers: tetrahydrofuran, diethyl ether, diisopropyl ether, 1,4 -dioxane, and the like, and glycol ethers, such as ethylene glycol monomethyl ether, diethylene glycol monomethyl ether, and propylene glycol monomethyl ether.

Aprotic polar solvents: N,N-dimethylformamide, N,N-dimethylacetamide, dimethyl sulfoxide, N-methyl-2-pyrrolidone, sulfolane, and the like.

Esters: ethyl acetate, isobutyl acetate, ethyl lactate, dimethyl phthalate, diethyl phthalate, ethylene carbonate, propylene carbonate, propylene glycol monomethyl ether acetate, and the like.

Hydrocarbons: petroleum ether, pentane, hexane, heptane, benzene, toluene, xylene, liquid paraffin, gasoline, and mineral oil.

Higher fatty acids: fatty acids with 4 or more (preferably 4 to 30) carbon atoms other than those in carboxyl groups, such as oleic acid, linoleic acid, and linolenic acid.

In particular, a preferred example of the organic solvent resistance of the nanofiltration membrane of the present invention is having resistance to at least one, preferably all of, the below-listed organic solvents:

Alcohols: isopropyl alcohol, benzyl alcohol, ethylene glycol, and glycerin.

Ketones: acetone, methyl ethyl ketone, and cyclohexanone.

Ethers: tetrahydrofuran, diethyl ether, and propylene glycol monomethyl ether.

Aprotic polar solvents: N,N-dimethylformamide, N,N-dimethylacetamide, dimethyl sulfoxide, and N-methyl-2-pyrrolidone.

Esters: ethyl acetate, isobutyl acetate, and dimethyl phthalate.

Hydrocarbons: hexane, heptane, benzene, toluene, gasoline, and mineral oil.

Higher fatty acids: oleic acid and linoleic acid.

The organic solvent resistance of the nanofiltration membrane of the present invention is specifically such that, for example, when immersed in the above-described organic solvent at 25°C for 14 hours, the change rate in the tensile strength and elongation of the nanofiltration membrane after immersion is ± 30% or less, and preferably less than ± 20%, compared to the tensile strength and elongation before immersion. Specifically, the change rate in tensile strength and elongation is calculated according to the following equation:

[Expression 5]

$$\text{Change rate (\%)} = \{(\text{tensile strength or elongation before immersion} - \text{tensile strength or elongation after immersion})/\text{tensile strength or elongation before immersion}\} \times 100$$

When the nanofiltration membrane is a hollow fiber membrane, the strength and elongation of the nanofiltration membrane are the values measured under the conditions described in the [Tensile Strength and Elongation] section above. When the nanofiltration membrane is a flat sheet membrane, the strength and elongation of the nanofiltration membrane are the values measured under the conditions described in the [Tensile Strength and Elongation] section above, except that a sample prepared by cutting the flat sheet membrane into a rectangular shape with a width of 10 mm and a length of 100 mm is used.

[Uses]

The nanofiltration membrane of the present invention is used as a filtration membrane for subjecting a fluid to be treated containing a solute or particles to filtration treatment, to separate or concentrate a nanoscale solute or particles. The solute or particles to be cut off from the fluid to be treated using the nanofiltration membrane of the present invention have a nanoscale size, with a molecular weight more than or equal to the above-described molecular weight cut-off.

Moreover, the nanofiltration membrane of the present invention has a high flux of permeate for an organic solvent, and thus, is suitable for use in organic solvent nanofiltration. Examples of types of the organic solvent in the fluid to be treated that is subjected to organic solvent nanofiltration specifically include, but are not limited to, those organic solvents mentioned in the [Organic Solvent Resistance] section above.

The nanofiltration membrane of the present invention may be used to perform nanofiltration, by incorporating the nanofiltration membranes of the present invention into a nanofiltration membrane module as described below.

3. Method for Producing Nanofiltration Membrane

While the method for producing the nanofiltration membrane of the present invention is not limited as long as it produces a nanofiltration membrane that satisfies the above-described molecular weight cut-off and methanol permeability, one preferred example is a method comprising the below-described first to third steps. The nanofiltration membrane of the present invention is difficult to obtain under production conditions employing a known and common method, i.e., thermally induced phase separation (TIPS) or non-solvent induced phase separation (NIPS) alone; whereas the method comprising the below-described first to third steps adopts the principles of both TIPS and NIPS, and can thereby efficiently produce the nanofiltration membrane of the present invention.

First step: preparing a dope solution by dissolving a polyamide resin at a concentration of 25% by mass or more in an organic solvent at a temperature of 100°C or more, the organic solvent having a boiling point of 150°C or more and incompatible with the polyamide resin at a temperature of less than 100°C.

Second step: extruding the dope solution in a predetermined shape into a coagulation bath at 100°C or less to solidify the polyamide resin into a membrane, wherein at least one surface of the dope solution extruded in the predetermined shape is contacted with a coagulation liquid containing polyethylene glycol with an average molecular weight of 400 to 1,000 and/or polypropylene glycol with an average molecular weight of 400 to 1,000 to form a nanofiltration membrane.

Third step: removing the coagulation liquid from the nanofiltration membrane formed in the second step.

Each of the first to third steps will be hereinafter described in detail.

[First Step]

In the first step, a dope solution is prepared by dissolving a polyamide resin at a concentration of 25% by mass or more in an organic solvent at a temperature of 100°C or more, the organic solvent having a boiling point of 150°C or more and incompatible with the polyamide resin at a temperature of less than 100°C.

Examples of the organic solvent having a boiling point of 150°C or more and incompatible with the polyamide resin at a temperature of less than 100°C include aprotic polar solvents, glycerin ethers, polyhydric alcohols, organic acids and organic acid esters, and higher alcohols. Specific examples of aprotic polar solvents include sulfolane, dimethylsulfone, dimethylsulfoxide, γ-butyrolactone, δ-valerolactone, ε-caprolactone, N,N-dimethylformamide, N,N-dimethylacetamide, N-methyl-2-pyrrolidone, ethylene carbonate, and propylene carbonate. Specific examples of glycerin ethers include diethylene glycol dimethyl ether, diethylene glycol diethyl ether, triethylene glycol dimethyl ether, diethylene glycol dibutyl ether, and tetraethylene glycol dimethyl ether. Specific examples of polyhydric alcohols include glycerin, ethylene glycol, diethylene glycol, triethylene glycol, propylene glycol, hexylene glycol, 1,3-butanediol, and polyethylene glycol (molecular weight: 100 to 10,000). Specific examples of organic acids and organic acid esters include dimethyl phthalate, diethyl phthalate, diisopropyl phthalate, dibutyl phthalate, butyl benzyl phthalate, methyl salicylate, oleic acid, palmitic acid, stearic acid, and lauric acid. From the viewpoint of obtaining a nanofiltration membrane with a higher strength, preferred among these organic solvents are aprotic polar solvents and polyhydric alcohols; more preferred are sulfolane, dimethylsulfone, γ-butyrolactone, δ-valerolactone, ε-caprolactone, propylene glycol, hexylene glycol, 1,3-butanediol, and polyethylene glycol (molecular weight: 100 to 600); still more preferred are sulfolane, dimethylsulfone, γ-butyrolactone, δ-valerolactone, and ε-caprolactone; and particularly preferred are sulfolane and dimethylsulfone. These organic solvents may be used alone or in combination. While a sufficient effect can be obtained by using one of these organic solvents alone, it may be possible to produce a more effective nanofiltration membrane by using a mixture of two or more of these organic solvents, because of a difference in the order of phase separation and structure.

While the concentration of the polyamide resin in the dope solution is not limited as long as it is 25% by mass or more, it is preferably 25 to 50% by mass, more preferably 25 to 40% by mass, and still more preferably 25 to 35% by mass. When the concentration of the polyamide resin in the dope solution satisfies the above-defined range, the nanofiltration membrane can be provided with excellent strength while satisfying the above-described molecular weight cut-off and methanol permeability. If the concentration of the polyamide resin in the dope solution is less than 25% by mass, there is a tendency for the molecular weight cut-off to be over 1,000, resulting in a failure to obtain a nanofiltration membrane.

Moreover, in the first step, when dissolving the polyamide resin in the organic solvent, the temperature of the solvent needs to be adjusted to 100°C or more. Specifically, the polyamide resin is preferably dissolved in the organic solvent at a temperature 10 to 50°C higher, preferably 20 to 40°C higher, than the phase separation temperature of the dope solution to be prepared. The phase separation temperature of the dope solution refers to the temperature at which liquid-liquid phase separation or solid-liquid phase separation due to crystal precipitation occurs by gradually cooling the mixture obtained by mixing the polyamide resin and the organic solvent at a sufficiently high temperature. The phase separation temperature may be measured using, for example, a microscope equipped with a hot stage.

In the first step, the temperature condition for dissolving the polyamide resin in the organic solvent may be set appropriately in the range of temperatures of 100°C or more as indicated above, according to the type of polyamide resin and the type of organic solvent used. The temperature condition is preferably 120 to 250°C, more preferably 140 to 220°C, and still more preferably 160 to 200°C.

The dope solution may also optionally contain fillers, thickeners, antioxidants, surface modifiers, lubricants, surfactants, and the like, to control the pore size or improve the performance of the nanofiltration membrane, for example.

The dope solution prepared in the first step is subjected to the second step while maintaining the temperature (that is, 100°C or more).

[Second Step]

The second step is the step of extruding the dope solution prepared in the first step in a predetermined shape into a coagulation bath at 100°C or less to solidify the polyamide resin into a membrane, wherein at least one surface of the dope solution extruded in the predetermined shape is contacted with a coagulation liquid (which may also be designated as "dense layer-forming coagulation liquid, hereinafter) containing polyethylene glycol with an average molecular weight of 400 to 1,000 and/or polypropylene glycol with an average molecular weight of 400 to 1,000 to form a nanofiltration membrane.

In the dope solution extruded in the second step, near the surface where the dope solution is contacted with the dense layer-forming coagulation liquid, non-solvent induced phase separation by solvent exchange proceeds more predominantly than thermally induced phase separation by cooling. This allows the formation of a dense layer with a structure denser than that in prior art polyamide membranes. Consequently, the nanofiltration membrane of the present invention can be imparted with the above-described molecular weight cut-off and methanol permeability.

To form the dense layer on only one surface of the nanofiltration membrane, in the second step, the dense layer-forming coagulation liquid may be contacted with one surface of the dope solution extruded in the predetermined shape, while a coagulation liquid (which may also be designated as "porous structure-forming coagulation liquid, hereinafter) having compatibility with the organic solvent used in the dope solution and having high affinity for the polyamide resin may be contacted with the other surface of the dope solution. Alternatively, to form the dense layer on both surfaces of the nanofiltration membrane, in the second step, the dense layer-forming coagulation liquid may be contacted with both surfaces of the dope solution extruded in the predetermined shape.

The average molecular weight of the polyethylene glycol and/or polypropylene glycol to be used as the dense layer-forming coagulation liquid is not limited as long as it satisfies the range of 400 to 1,000; however, from the viewpoint of satisfactorily imparting the above-described molecular weight cut-off and methanol permeability, the average molecular weight of the polyethylene glycol and/or polypropylene glycol is preferably 400 to 800, and more preferably 400 to 600. If polyethylene glycol and/or polypropylene glycol with an average molecular weight of less than 400 is used, there is a tendency for the molecular weight cut-off to be over 1,000, resulting in a failure to obtain a nanofiltration membrane. As used herein, the average molecular weight of the polyethylene glycol and/or polypropylene glycol is the number average molecular weight calculated based on the hydroxyl number measured according to JIS K 1557 -6: 2009 "Plastics-Polyols for use in the production of polyurethanes- Part 6: Determination of hydroxyl number by NIR (Near-Infrared) spectroscopy".

Specific examples of the polyethylene glycol and/or polypropylene glycol to be used as the dense layer-forming coagulation liquid include polyethylene glycol 400, polyethylene glycol 600, polyethylene glycol 800, polyethylene glycol 1000, and polypropylene glycol 400. Preferred among these are polyethylene glycol 400, polyethylene glycol 600, and polypropylene glycol 400.

The dense layer-forming coagulation liquid may be formed using only one of the polyethylene glycol with the predetermined average molecular weight and the polypropylene glycol with the predetermined average molecular weight, or using the polyethylene glycol with the predetermined average molecular weight and the polypropylene glycol with the predetermined average molecular weight in combination.

While the dense layer-forming coagulation liquid is preferably formed of the polyethylene glycol and/or polypropylene glycol, it may also contain water in addition to the polyethylene glycol and/or polypropylene glycol, as long as it can satisfactorily impart the above-described molecular weight cut-off and methanol permeability. When the dense layer-forming coagulation liquid contains water, the water content is, for example, 80% by mass or less, preferably 40% by mass or less, more preferably 20% by mass or less, still more preferably 10% by mass or less, and particularly preferably 5% by mass or less.

The porous structure-forming coagulation liquid may be any solvent that is compatible with the organic solvent used in the dope solution at a temperature of 25 °C or less, and dissolves the polyamide resin at a temperature less than or equal to the boiling point. Specific examples of the porous structure-forming coagulation liquid include glycerin, ethylene glycol, diethylene glycol, triethylene glycol, tetraethylene glycol, polyethylene glycol with an average molecular weight of 200 to 800, propylene glycol, 1,3-butanediol, sulfolane, N-methyl-2-pyrrolidone, γ-butyrolactone, δ-valerolactone, and aqueous solutions containing 20% by mass or more of these solvents. Among these, preferred are at least one selected from the group consisting of glycerin, propylene glycol, diethylene glycol, and polyethylene glycol with an average molecular weight of 200 to 600, as well as aqueous solutions containing 20 to 75% (preferably 25 to 75% by mass) by mass of these solvents; more preferred are at least one selected from the group consisting of glycerin, diethylene glycol, tetraethylene glycol, and propylene glycol, as well as aqueous solutions containing 40 to 80% by mass (preferably 40 to 60% by mass) of at least one of these solvents, or alternatively, polyethylene glycol with an average molecular weight of 200 to 600 and an aqueous solution containing 20 to 75% by mass of polyethylene glycol with an average molecular weight of 200 to 600; and particularly preferred are propylene glycol and an aqueous solution containing 40 to 80% by mass (preferably 40 to 60% by mass) of propylene glycol.

When a hollow fiber membrane is to be formed as the nanofiltration membrane, in the second step, a double-tube nozzle for hollow fiber production with a double-tube structure may be used to discharge the dope solution from an outer annular nozzle while simultaneously discharging an internal coagulation liquid from an inner nozzle to immerse the dope solution and the internal coagulation liquid in a coagulation bath. In this case, the dense layer-forming coagulation liquid may be used as at least one of the internal coagulation liquid and the coagulation bath. When the dense layer-forming coagulation liquid is used as both the internal coagulation liquid and the coagulation bath, a hollow fiber membrane is obtained in which the dense layer is formed on both the lumen-side surface and the outer surface, and the inside is formed of porous regions. Alternatively, when the dense layer-forming coagulation liquid is used as the internal coagulation liquid, and the porous structure-forming coagulation liquid is used as the coagulation bath, a hollow fiber membrane is obtained in which the dense layer is formed on the lumen-side surface, and the inside and the outer surface are formed of porous regions. Alternatively, when the porous structure-forming coagulation liquid is used as the internal coagulation liquid, and the dense layer-forming coagulation liquid is used as the coagulation bath, a hollow fiber membrane is obtained in which the dense layer is formed on the outer surface, and the lumen-side surface and the inside are formed of porous regions. Since the internal coagulation liquid used for forming the hollow fiber membrane passes through the double-tube nozzle, it preferably does not contain water having a boiling point less than or equal to the temperature of the double-tube nozzle.

The double-tube nozzle for hollow fiber production may be a spinneret having a double-tube structure, such as that used in the production of core-sheath composite fibers by melt spinning. The diameter of the outer annular nozzle and the diameter of the inner nozzle of the double-tube nozzle for hollow fiber production may be set appropriately according to the inner and outer diameters of the hollow fiber membrane.

The flow rate of the dope solution when discharged from the outer annular nozzle of the double-tube nozzle for hollow fiber production varies with the slit width and thus, is not limited; for example, it is 2 to 30 g/min, preferably 3 to 20 g/min, and more preferably 5 to 15 g/min. The flow rate of the internal coagulation fluid is set appropriately in consideration of the diameter of the inner nozzle of the double-tube nozzle for hollow fiber production, the type of internal liquid used, the flow rate of the dope solution, and the like; for example, it is 0.1 to 2 times, preferably 0.2 to 1 times, and more preferably 0.4 to 0.7 times the flow rate of the dope solution.

When a flat sheet membrane is to be formed as the nanofiltration membrane, in the second step, the dense layer-forming coagulation liquid may be used as the coagulation bath, and the dope solution may be extruded in a predetermined shape into the coagulation bath and immersed therein.

In the second step, the temperature of the coagulation bath may be any temperature that is 100°C or less; preferably, it is -20 to 100°C, more preferably 0 to 60°C, still more preferably 2 to 20°C, and particularly preferably 2 to 10°C. The preferred temperature of the coagulation bath may vary depending on the organic solvent used in the dope solution, the composition of the coagulation liquid, and the like; however, in general, thermally induced phase separation tends to proceed preferentially when the coagulation bath has a lower temperature, and non-solvent induced phase separation tends to proceed preferentially when the coagulation bath has a higher temperature. That is, when producing a hollow fiber membrane in which the dense layer is formed on the lumen-side surface, it is preferred to set the coagulation bath at a lower temperature to increase the pore size in the dense layer on the lumen-side surface, while it is preferred to set the coagulation bath at a higher temperature to further densify the denser layer on the lumen-side surface, and make the internal structure coarse.

When a hollow fiber membrane is to be formed as the nanofiltration membrane, the temperature of the internal coagulation liquid may be about the set temperature of the double-tube nozzle, for example, 120 to 250°C, preferably 160 to 230°C, and more preferably 180 to 220°C.

By performing the second step as described above, the dope solution is solidified in the coagulation bath, and simultaneously, a nanofiltration membrane is formed having a dense layer formed on at least one surface.

[Third Step]

In the third step, the coagulation liquid is removed from the nanofiltration membrane formed in the second step. While the method of removing the coagulation liquid from the nanofiltration membrane is not limited, it is preferably a method in which the nanofiltration membrane is immersed in an extraction solvent to extract and remove the coagulation liquid undergoing phase separation in the nanofiltration membrane. The extraction solvent to be used in the extraction and removal of the organic solvent is preferably a solvent that is inexpensive, and has a low boiling point and can be readily separated after the extraction by utilizing a difference in boiling point or the like. Examples of the extraction solvent include water, glycerin, methanol, ethanol, isopropanol, acetone, diethyl ether, hexane, petroleum ether, and toluene. Preferred among these are water, methanol, ethanol, isopropanol, and acetone; and more preferred are water, methanol, and isopropanol. In particular, for extraction of the coagulation liquid that dissolves in water, it is efficient to take up the nanofiltration membrane while showering it with water, which allows the solvent extraction to take place simultaneously. For extraction of a water-insoluble organic solvent, such as a phthalic acid ester or a fatty acid, isopropyl alcohol, petroleum ether, or the like can be suitably used.

When the coagulation liquid is extracted and removed by immersing the nanofiltration membrane in the extraction solvent, the time for immersing the nanofiltration membrane in the extraction solvent is not limited but is, for example, 0.2 hour to 2 months, preferably 0.5 hour to 1 month, and still more preferably 2 hours to 10 days. For effective extraction and removal of the coagulation liquid remaining in the nanofiltration membrane, the extraction solvent may be replaced or stirred.

By performing the third step as described above, the nanofiltration membrane of the present invention is obtained.

[Drying Step (Fourth Step)]

The method for producing the nanofiltration membrane of the present invention preferably includes the fourth step of drying and removing the extraction solvent from the nanofiltration membrane after the third step. The drying and removal of the extraction solvent may be performed using a known drying treatment, such as natural drying, hot-air drying, reduced pressure drying, or vacuum drying.

Moreover, simultaneously with the treatment of drying and removing the extraction solvent or after the drying treatment, the nanofiltration membrane may be uniaxially stretched. By uniaxially stretching the nanofiltration membrane, it is possible to improve the tensile strength and elongation while increasing the methanol permeability while maintaining the above-defined range of the molecular weight cut-off. To uniaxially stretch the nanofiltration membrane simultaneously with the drying and removal treatment, the nanofiltration membrane may be subjected to the drying and removal treatment while tension for the stretching is being applied. While the mechanism by which the methanol permeability can be increased while maintaining the above-defined range of the molecular weight cut-off by uniaxially stretching the nanofiltration membrane is not fully clear, it is believed, for example, as follows: It is assumed that the stretching causes a pore present in the dense layer to expand in the form of an ellipse, at which time the minor diameter of the ellipse maintains the original diameter of the pore, such that the molecular weight cut-off is maintained, while the area of the entire ellipse becomes larger than the area of the original pore, resulting in achievement of the above-described effect.

The stretching ratio for the uniaxial stretching of the nanofiltration membrane is, for example, 1.2 to 5 times, and preferably 1.2 to 3 times. The stretching may be performed using a known method, for example, it may be performed continuously by taking up the nanofiltration membrane from a low-speed roll to a high-speed roll. Alternatively, the stretching may be performed with a tensile testing machine or the like while holding both ends of the membrane cut to a certain length, or may be performed by manual stretching.

4. Nanofiltration Membrane Module

The nanofiltration membranes of the present invention are housed in a module casing with an inlet for the fluid to be treated, an outlet for the permeate, and the like, and used as a nanofiltration membrane module.

When the nanofiltration membranes of the present invention are in the form of hollow fibers, the nanofiltration membranes are used as a hollow fiber membrane module.

Specifically, the hollow fiber membrane module may be any module configured such that a bundle of the hollow fiber-shaped nanofiltration membranes of the present invention is housed in the module casing, and one or both ends of the hollow fiber-shaped nanofiltration membranes are sealed with a potting material and anchored. The hollow fiber membrane module may be any module having an opening connected to a flow passage passing the outer wall side of the hollow fiber-shaped nanofiltration membranes and an opening connected to the hollow portions of the hollow fiber-shaped nanofiltration membranes, as an inlet for the fluid to be treated or an outlet for the filtrate.

The hollow fiber membrane module is not limited in shape, and may be a dead-end module or a crossflow module. Specific examples of shapes of the hollow fiber membrane module include a dead-end module produced by packing a hollow fiber membrane bundle bent in a U-shape, and sealing ends of the hollow fiber-shaped nanofiltration membrane bundle and then cutting the ends to make them open; a dead-end module produced by packing straight a hollow fiber-shaped nanofiltration membrane bundle whose hollow opening at one end is closed by heat sealing or the like, and sealing an open end of the hollow fiber-shaped nanofiltration membrane bundle and then cutting the end to make it open; a dead-end module produced by packing straight a hollow fiber-shaped nanofiltration membrane bundle, and sealing the ends of the hollow fiber-shaped nanofiltration membrane bundle and then cutting only one end to make the opening exposed; and a crossflow module produced by packing straight a hollow fiber-shaped nanofiltration membrane bundle, sealing the ends of the hollow fiber-shaped nanofiltration membrane bundle, cutting the sealed portions at the ends of the hollow fiber-shaped nanofiltration membrane bundle, and creating two flow passages on the side surface of the filter case.

While the packing ratio of the hollow fiber-shaped nanofiltration membranes inserted into the module casing is not limited, it may be such that, for example, the volume of the hollow fiber-shaped nanofiltration membranes including the volume of the hollow portions to the internal volume of the module casing is 30 to 90% by volume, preferably 35 to 75% by volume, and more preferably 45 to 65% by volume. When the packing ratio satisfies these ranges, it is possible to facilitate the operation of packing the hollow fiber-shaped nanofiltration membranes into the module casing, and facilitate the flow of the potting material between the hollow fiber-shaped nanofiltration membranes, while ensuring a sufficient filtration area.

While the potting material used to produce the hollow fiber membrane module is not limited, it preferably contains an organic solvent resistance when the hollow fiber membrane module is for use in the treatment of an organic solvent, and examples of such potting agents include polyamides, silicone resins, epoxy resins, melamine resins, polyethylenes, polypropylenes, phenolic resins, polyimides, and polyurea resins. Preferred among these potting materials are those with low shrinkage or swelling upon curing, and a hardness that is not excessively high. Preferred examples of potting materials include polyamides, silicone resins, epoxy resins, and polyethylenes. These potting materials may be used alone or in combination.

Examples of materials of the module casing used for the hollow fiber membrane module include, but are not limited to, polyamides, polyesters, polyethylenes, polypropylenes, polyvinylidene fluorides, polytetrafluoroethylenes, polyvinyl chlorides, polysulfones, polyethersulfones, polycarbonates, polyarylates, and polyphenylene sulfides. Preferred among these are polyamides, polyethylenes, polypropylenes, polytetrafluoroethylenes, polycarbonates, polysulfones, and polyethersulfones, and more preferred are polyamides, polyethylenes, polypropylenes, and polytetrafluoroethylenes.

When the nanofiltration membranes of the present invention are in the form of flat sheet membranes, the nanofiltration membranes are used as a sheet-type module, such as a plate-and-frame-type or a stacked-type module, a spiral-type module, a rotating flat sheet membrane-type module, or the like.

The nanofiltration membrane module produced using the nanofiltration membranes of the present invention is used in fields such as the semiconductor industry, chemical industry, food industry, pharmaceutical industry, and medical goods industry, for removal of foreign matter in solvents, concentration of useful components in solvents, solvent recovery, water purification, and the like. In one embodiment, the nanofiltration membrane module produced using the nanofiltration membranes of the present invention is suitable for use in organic solvent nanofiltration.

Examples

The present invention will be hereinafter described in more detail with examples; however, the present invention is not limited to these examples.

1. Measurement Methods

[Outer and Inner Diameters of Hollow Fiber Membrane and Thickness of Hollow Fiber Membrane]

Five hollow fiber membranes were observed with an optical microscope at 200x magnification, the outer and inner diameters (both at the point of the maximum diameter) of each hollow fiber membrane were measured, and the average value of each of the outer and inner diameters was determined. The thickness of the hollow fiber membrane was calculated by dividing the outer diameter minus the inner diameter by 2.

[Thickness of Dense Layer]

A cross section of the hollow fiber membrane [previously subjected to vapor deposition treatment with platinum at a discharge voltage of 45 mA for a vapor deposition time of 15 seconds, using a vapor deposition apparatus (MSP-1S-type magnetron sputtering apparatus available from VACUUM DEVICE)] was observed with a scanning electron microscope (SEM) at 10,000x magnification, distances (thicknesses) of 10 or more regions where substantially no pores were observed to be present were measured, and the average value of the measurements was determined.

[Methanol Permeability]

First, a module as shown in Fig. 1a was produced. Specifically, 10 hollow fiber membranes were cut into a length of 30 cm, aligned and bundled to prepare bundled hollow fiber membranes. Next, a rigid nylon tube with an outer diameter of 8 mm, an inner diameter of 6 mm, and a length of 50 mm was prepared, and, through one end opening of the tube, a rubber stopper with a length of about 20 mm was inserted to plug the one end opening. Next, a two-part mixture, room temperature curable epoxy resin was inserted into the opening opposite the opening with the rubber stopper to fill the inner space of the tube with the epoxy resin. Then, the bundled hollow fiber membranes prepared above were bent in a substantially U-shape, and both ends of the hollow fiber membranes were inserted into the tube filled with the epoxy resin, until the tips of the ends touched the rubber stopper. In this state, the epoxy resin was allowed to cure. Then, the rubber stopper-side region of the cured epoxy resin portions was cut together with the tube to produce a module in which the hollow portions at both ends of the hollow fiber membranes were open.

Next, the module was mounted on the apparatus as shown in Fig. 1b, and methanol (100% methanol) at 25°C was passed through the inside of the hollow fiber membranes of the module at a pressure of about 0.3 MPa for a certain time. The volume of methanol permeated out of the hollow fiber membranes was determined, and the methanol permeability ($L/(m^2 \cdot bar \cdot h)$) was calculated according to the following equation:

[Expression 6]

$$\text{Methanol permeability} = \text{volume (L) of methanol permeated out of the hollow fiber membranes}/[\text{inner diameter (m) of the hollow fiber membranes} \times 3.14 \times \text{effective filtration length (m) of the hollow fiber membranes} \times 10 \text{ (number of membranes)} \times \{[\text{pressure (bar)}\} \times \text{time (h)}]$$

[Molecular Weight Cut-Off]

Using a 0.1% by mass solution of a commercial polyethylene glycol standard for GPC (PEG; Agilent Technologies, molecular weight: 194, 238, 282, 420, 600, 1,000, 1,500 or 4,000) in methanol as the feed fluid, the feed fluid was passed at a pressure of 0.3 MPa, the permeated fluid was collected, the polyethylene glycol concentration in the permeate was measured by high performance liquid chromatography, and the rejection rate was calculated according to the equation shown below. Based on the result of the rejection rate for the polyethylene glycol of each molecular weight, a graph was plotted in which the horizontal axis represents the molecular weights of the polyethylene glycols used and the vertical axis represents the rejection rates, and the molecular weight at the intersection of the resulting approximate curve and the 90% rejection rate was determined as the molecular weight cut-off.

[Expression 7]

$$\text{Rejection rate (\%)} = \{(\text{concentration of the polyethylene glycol in the feed fluid} - \text{concentration of the polyethylene glycol in the permeate})/\text{concentration of the polyethylene glycol in the feed fluid}\} \times 100$$

[Tensile Strength and Elongation]

A sample prepared by cutting the hollow fiber membrane into a length of about 10 cm was subjected to a tensile test at a grip distance of 50 mm and a tensile speed of 50 mm/min, in an environment at a room temperature of 25 °C and a humidity of 60%, to measure the load (N) and the elongation (mm) at break. Separately, a cross section of the hollow fiber membrane was observed with an optical microscope at 200x magnification, the outer and inner diameters (both at the point of the maximum diameter) of the hollow fiber membrane were measured, and the cross-sectional area ($mm^2$) of the hollow fiber membrane was determined from the outer and inner diameters obtained. The tensile test and the measurement of the cross-sectional area were performed using five hollow fiber membranes, the average value of each of the load at break, the elongation at break, and the cross-sectional area was calculated, and the tensile strength and elongation were calculated using these average values, according to the following equations:

[Expression 8]

$$\text{Tensile strength (MPa)} = \text{load (N) at break/cross-sectional area (mm}^2\text{) of the}$$

$$\text{hollow fiber membrane}$$

$$\text{Elongation (\%)} = (\text{elongation (mm) at break/grip distance (mm))} \times 100$$

[Organic Solvent Resistance]

The hollow fiber membrane was immersed in various organic solvents at 25°C for 14 days. The tensile strength and elongation of the polyamide hollow fiber membrane were measured under the above-described conditions before and after immersion, and the change rate was calculated according to the following equation:

[Expression 9]

$$\text{Change Rate (\%)} = \{(\text{tensile strength or elongation before immersion} - \text{tensile}$$

$$\text{strength or elongation after immersion)/tensile strength or elongation before}$$

$$\text{immersion}\} \times 100$$

2. Production of Hollow Fiber Membranes

[Example 1]

260 g of polyamide 6 chips (A1030BRT available from UNITIKA LTD., relative viscosity: 3.53) and 740 g of sulfolane (available from Tokyo Chemical Industry) were stirred at 180°C for 1.5 hours to dissolve the chips, and the solution was degassed for 1 hour at a reduced stirring rate to prepare a dope solution. The dope solution was fed to a spinneret maintained at a temperature of 210°C through a metering pump, and extruded at 13.0 g/min. The spinneret had an outer diameter of 1.5 mm and an inner diameter of 0.6 mm. As an internal coagulation liquid (dense layer-forming coagulation liquid), polyethylene glycol 400 (PEG400, average molecular weight: 400) was passed at a feed rate of 5.0 g/min. The extruded dope solution was introduced through an air gap of 10 mm into a coagulation bath of a 50% by mass aqueous solution of propylene glycol (PG) (porous structure-forming coagulation liquid) at 5°C, cooled and solidified, and then taken up at a take-up rate of 20 m/min. The resulting polyamide hollow fiber was immersed in water for 24 hours to extract the solvent, and then dried by passing through a hot-air dryer (chamber temperature: 130°C) without stretching. Thus, a polyamide hollow fiber membrane was obtained.

[Example 2]

A polyamide hollow fiber membrane was obtained under the same conditions as in Example 1, except that the dope solution was prepared using 320 g of polyamide 6 chips and 680 g of sulfolane.

[Example 3]

A polyamide hollow fiber membrane was produced under the same conditions as in Example 2, except that the internal coagulation liquid (dense layer-forming coagulation liquid) was changed to polyethylene glycol 600 (PEG600, average molecular weight: 600).

[Example 4]

A polyamide hollow fiber membrane was produced under the same conditions as in Example 2, except that the internal coagulation liquid (dense layer-forming coagulation liquid) was changed to polypropylene glycol 400 (PPG400, average molecular weight: 400).

[Example 5]

A polyamide hollow fiber membrane was produced under the same conditions as in Example 2, except that the dope solution was prepared using 320 g of polyamide 6 chips, 544 g of dimethyl sulfone, and 136 g of sulfolane.

[Example 6]

A polyamide hollow fiber membrane was obtained under the same conditions as in Example 3, except that the coagulation bath was changed to a 20% by mass aqueous solution of polyethylene glycol 600 (PEG600, average molecular weight: 600) (porous structure-forming coagulation liquid).

[Example 7]

A polyamide hollow fiber membrane was obtained under the same conditions as in Example 1, except that the dope solution was prepared using 250 g of polyamide 11 chips (Rilsan BESV0 A FDA available from Arkema, relative viscosity: 2.50) and 750 g of γ-butyrolactone (available from Wako Pure Chemical Corporation).

[Example 8]

A polyamide hollow fiber membrane was produced under the same conditions as in Example 6, except that the hollow fiber membrane taken up after being cooled and solidified was immersed in water for 24 hours to extract the solvent, and then dried and stretched (stretching ratio: 2 times) simultaneously by sequentially passing through a feed roller, a hot-air dryer (chamber temperature: 130°C), and a take-up (stretching) roller.

[Comparative Example 1]

A polyamide hollow fiber membrane was produced under the same conditions as in Example 2, except that the internal coagulation liquid (dense layer-forming coagulation liquid) was changed to polyethylene glycol 300 (PEG300, average molecular weight: 300).

[Comparative Example 2]

A polyamide hollow fiber membrane was obtained under the same conditions as in Example 1, except that the dope solution was prepared using 240 g of polyamide 6 chips and 760 g of sulfolane.

3. Evaluation Results of Physical Properties of Hollow Fiber Membranes

In each of the polyamide hollow fiber membranes of Examples 1 to 8 and Comparative Examples 1 and 2, a dense layer was formed on the lumen-side surface. Table 1 shows the measured results of the outer diameter, the inner diameter, the thickness of the hollow fiber membrane, the thickness of the dense layer, the methanol permeability, the molecular weight cut-off, the tensile strength, and the elongation for each polyamide hollow fiber membrane.
The polyamide hollow fiber membranes (Examples 1 to 8), each produced using a dope solution with a resin concentration of 25% by mass or more, and using polyethylene glycol or polypropylene glycol with an average molecular weight of 400 or more and 1,000 or less as the internal coagulation liquid, had a high flux of permeate, i.e., a methanol permeability of 0.03 L/(m$^2$·bar·h) or more, while having a molecular weight cut-off of 200 to 1,000. In contrast, the polyamide hollow fiber membrane (Comparative Example 1), produced using a dope solution with a resin concentration of 25% by mass or more, but using polyethylene glycol with an average molecular weight of 300 as the internal coagulation liquid, had a molecular weight cut-off as high as 1,800, and was not usable in nanofiltration. Moreover, the polyamide hollow fiber membrane (Comparative Example 2), produced using polyethylene glycol with an average molecular weight of 400 or more and 1,000 or less as the internal coagulation liquid, but using a dope solution with a resin concentration of 24% by mass, had a molecular weight cut-off as high as 1,300, and was not usable in nanofiltration.

[Table 1]

| | Resin Used | Production Conditions | | | | Physical Properties of Hollow Fiber Membrane | | | | | | | |
|---|---|---|---|---|---|---|---|---|---|---|---|---|---|
| | | Dope Solution | | Internal Coagulation Liquid | Coagulation Bath | Outer Diameter ($\mu$m) | Inner Diameter ($\mu$m) | Thickness ($\mu$m) of Hollow Fiber Membrane | Thickness (nm) of Dense Layer | Methanol Permeability (L/m$^2$·bar·h) | Molecular Weight Cut-Off (Da) | Tensile Strength (MPa) | Elongation (%) |
| | | Resin Concentration (% by Mass) | Solvent | | | | | | | | | | |
| Ex. 1 | Ny6 | 26 | Sulfolane | PEG400 | 50 % by Mass PG | 960 | 580 | 190 | 720 | 1.20 | 990 | 11.8 | 230 |
| Ex. 2 | Ny6 | 32 | Sulfolane | PEG400 | 50 % by Mass PG | 980 | 590 | 195 | 800 | 0.72 | 760 | 15.2 | 170 |
| Ex. 3 | Ny6 | 32 | Sulfolane | PEG600 | 50 % by Mass PG | 960 | 570 | 195 | 680 | 0.49 | 600 | 14.8 | 170 |
| Ex. 4 | Ny6 | 32 | Sulfolane | PPG400 | 50 % by Mass PG | 500 | 300 | 100 | 320 | 0.88 | 880 | 14.4 | 200 |
| Ex. 5 | Ny6 | 32 | Dimethyl Sulfone + Sulfolane | PEG400 | 50 % by Mass PG | 1980 | 1260 | 360 | 930 | 0.92 | 820 | 14.9 | 140 |
| Ex. 6 | Ny6 | 32 | Sulfolane | PEG600 | 20 % by Mass PEG600 | 980 | 580 | 200 | 730 | 0.10 | 280 | 14.9 | 160 |
| Ex. 7 | Ny11 | 25 | $\gamma$-Butyrolactone | PEG400 | 50 % by Mass PG | 1420 | 810 | 305 | 870 | 0.37 | 910 | 13.2 | 150 |
| Ex. 8 | Ny6 | 32 | Sulfolane | PEG600 | 20 % by Mass PEG600 | 790 | 370 | 210 | 440 | 0.41 | 620 | 29.3 | 120 |
| Comp. Ex. 1 | Ny6 | 32 | Sulfolane | PEG300 | 50 % by Mass PG | 970 | 580 | 195 | 660 | 0.49 | 1800 | 14.2 | 180 |
| Comp. Ex. 2 | Ny6 | 24 | Sulfolane | PEG400 | 50 % by Mass PG | 990 | 590 | 200 | 820 | 1.50 | 1300 | 14.6 | 170 |

In the table, Ny6 is an abbreviation of polyamide 6, and Ny11 is an abbreviation of polyamide 11.

4. Organic Solvent Resistance

Table 2 shows the measured results of the organic solvent resistance for the hollow fiber membranes of Examples 1 and 7. These results have confirmed that the hollow fiber membranes of Examples 1 and 7 have resistance to a wide range of organic solvents. Since the hollow fiber membranes of Examples 2 to 6 and 8 are also formed of polyamide as with the hollow fiber membranes of Examples 1 and 7, it is clear from these results that the hollow fiber membranes of Examples 2 to 6 and 8 also have excellent organic solvent resistance.

[Table 2]

| Classification | Organic Solvents | Example 1 (Formed with Ny6) Change (Strength/Elongation)# | Example 7 (Formed with Ny11) Change (Strength/Elongation)# |
|---|---|---|---|
| Alcohols | Isopropyl Alcohol | ○/○ | ○/○ |
| | Benzyl Alcohol | ○/○ | ○/○ |
| | Ethylene Glycol | ○/○ | ○/○ |
| | Glycerin | ○/○ | ○/○ |
| Ketones | Acetone | ○/○ | ○/○ |
| | Methyl Ethyl Ketone | ○/○ | ○/○ |
| | Cyclohexanone | ○/○ | ○/○ |
| Ethers | Tetrahydrofuran | ○/○ | ○/○ |
| | Diethyl Ether | ○/○ | ○/○ |
| | Propylene Glycol Monomethyl Ether | ○/○ | ○/○ |
| Aprotic Polar Solvents | N,N-Dimethylformamide | ○/○ | ○/○ |
| | N,N-Dimethylacetamide | ○/○ | ○/○ |
| | Dimethyl Sulfoxide | ○/○ | ○/○ |
| | N-Methyl-2-Pyrrolidone | ○/○ | ○/○ |
| Esters | Ethyl Acetate | ○/○ | ○/○ |
| | Isobutyl Acetate | ○/○ | ○/○ |
| | Dimethyl Phthalate | ○/○ | ○/○ |
| | Ethylene Carbonate | ○/○ | ○/○ |
| Hydrocarbons | Hexane | ○/○ | ○/○ |
| | Heptane | ○/○ | ○/○ |
| | Benzene | ○/○ | ○/○ |
| | Toluene | ○/○ | ○/○ |
| | Gasoline | ○/○ | ○/○ |
| | Mineral Oil | ○/○ | ○/○ |
| Fatty Acids | Oleic Acid | ○/○ | ○/○ |
| | Linoleic Acid | ○/○ | ○/○ |
| #: The results of the change were classified as ○ when the change rate was less than 20%, △ when the change rate was 20% or more and 30% or less, and × when the change rate was above 30%. | | | |

Reference Signs List

1: module
1a: hollow fiber membranes
1b: tube filled with cured epoxy resin
2: feed pump
3: pressure gauge
4: pressure relief valve
5: receiving tray
6: methanol permeated out of hollow fiber membranes

**Claims**

1.  A nanofiltration membrane formed using a polyamide resin, the nanofiltration membrane having a molecular weight cut-off of 200 to 1,000 and a methanol permeability of 0.03 L/(m$^2$·bar·h) or more.

2.  The nanofiltration membrane according to claim 1, which has a molecular weight cut-off of 250 to 990.

3.  The nanofiltration membrane according to claim 1, which is a hollow fiber membrane with an outer diameter of 450 $\mu$m or more.

4.  The nanofiltration membrane according to any one of claims 1 to 3, wherein the polyamide resin consists of only one aliphatic polyamide resin having methylene and amide groups at a molar ratio of -CH$_2$- : -NHCO- = 4:1 to 10:1.

5.  The nanofiltration membrane according to any one of claims 1 to 4, wherein the polyamide resin is polyamide 6.

6.  The nanofiltration membrane according to any one of claims 1 to 5, which is used for organic solvent nanofiltration.

7.  A nanofiltration method comprising subjecting a fluid to be treated containing a solute or particles to filtration treatment, using the nanofiltration membrane according to any one of claims 1 to 6.

8.  The nanofiltration method according to claim 7, wherein a solvent contained in the fluid to be treated is an organic solvent.

9.  A nanofiltration membrane module comprising the nanofiltration membrane according to any one of claims 1 to 6, the nanofiltration membrane being housed in a module casing.

10. A method for producing a nanofiltration membrane comprising the following first to third steps:

    the first step of preparing a dope solution by dissolving a polyamide resin at a concentration of 25% by mass or more in an organic solvent at a temperature of 100°C or more, the organic solvent having a boiling point of 150°C or more and incompatible with the polyamide resin at a temperature of less than 100°C;
    the second step of extruding the dope solution in a predetermined shape into a coagulation bath at 100°C or less to solidify the polyamide resin into a membrane, wherein at least one surface of the dope solution extruded in the predetermined shape is contacted with a coagulation liquid containing polyethylene glycol with an average molecular weight of 400 to 1,000 and/or polypropylene glycol with an average molecular weight of 400 to 1,000 to form a nanofiltration membrane; and
    the third step of removing the coagulation liquid from the nanofiltration membrane formed in the second step.

11. The method for producing a nanofiltration membrane according to claim 10, which is a method for producing the nanofiltration membrane in the form of a hollow fiber membrane,

    wherein the second step is the step of using a double-tube nozzle for hollow fiber production with a double-tube structure to discharge the dope solution from an outer annular nozzle while simultaneously discharging an internal coagulation liquid from an inner nozzle to immerse the dope solution and the internal coagulation liquid in a coagulation bath, and
    a coagulation liquid containing polyethylene glycol with an average molecular weight of 400 to 1,000 and/or polypropylene glycol with an average molecular weight of 400 to 1,000 is used as at least one of the internal coagulation liquid and the coagulation bath.

12. The method for producing a nanofiltration membrane according to claim 10 or 11, which comprises the step of uniaxially stretching the nanofiltration membrane after the third step simultaneously with or after a drying treatment.

FIG. 1

## INTERNATIONAL SEARCH REPORT

| International application No. |
|---|
| **PCT/JP2021/035114** |

### A. CLASSIFICATION OF SUBJECT MATTER

*B01D 63/02*(2006.01)i; *B01D 69/02*(2006.01)i; *B01D 69/06*(2006.01)i; *B01D 71/56*(2006.01)i; *D01F 6/60*(2006.01)i
FI: B01D71/56; B01D69/06; B01D63/02; B01D69/02; D01F6/60 301Z

According to International Patent Classification (IPC) or to both national classification and IPC

### B. FIELDS SEARCHED

Minimum documentation searched (classification system followed by classification symbols)

B01D63/02; B01D69/02; B01D69/06; B01D71/56; D01F6/60

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); Japio-GPG/FX

### C. DOCUMENTS CONSIDERED TO BE RELEVANT

| Category* | Citation of document, with indication, where appropriate, of the relevant passages | Relevant to claim No. |
|---|---|---|
| X | WO 2014/030585 A1 (UNITIKA LTD) 27 February 2014 (2014-02-27) paragraphs [0001]-[0099], fig. 1-5 | 1-12 |
| X | JP 2014-036946 A (UNITIKA LTD) 27 February 2014 (2014-02-27) paragraphs [0001]-[0064], fig. 1-4 | 1-12 |
| X | JP 2019-526431 A (ENTEGRIS, INC) 19 September 2019 (2019-09-19) claims 1-20, paragraphs [0001]-[0088], fig. 1-4 | 1-12 |
| A | KR 10-2020-0075347 A (SYNOPEX CO LTD) 26 June 2020 (2020-06-26) claims 1-11 | 1-12 |

☐ Further documents are listed in the continuation of Box C.     ☑ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | "X" | document of particular relevance; the claimed invention cannot be considered novel or cannot be considered to involve an inventive step when the document is taken alone |
| "E" earlier application or patent but published on or after the international filing date | | |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" | document member of the same patent family |
| "P" document published prior to the international filing date but later than the priority date claimed | | |

| Date of the actual completion of the international search | Date of mailing of the international search report |
|---|---|
| **18 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/035114**

| Patent document cited in search report | | | Publication date (day/month/year) | Patent family member(s) | | | Publication date (day/month/year) |
|---|---|---|---|---|---|---|---|
| WO | 2014/030585 | A1 | 27 February 2014 | US | 2015/0209735 | A1 | |
| | | | | paragraphs [0001]-[0155], fig. 1-5 | | | |
| | | | | EP | 2889078 | A1 | |
| | | | | KR | 10-2015-0046138 | A | |
| | | | | CN | 104640619 | A | |
| | | | | TW | 201417998 | A | |
| JP | 2014-036946 | A | 27 February 2014 | (Family: none) | | | |
| JP | 2019-526431 | A | 19 September 2019 | US | 2019/0255489 | A1 | |
| | | | | claims 1-20, paragraphs [0001]-[0157], fig. 1-4 | | | |
| | | | | WO | 2018/005326 | A1 | |
| | | | | EP | 3474973 | A1 | |
| | | | | TW | 201811422 | A | |
| | | | | KR | 10-2019-0020799 | A | |
| | | | | CN | 109562324 | A | |
| KR | 10-2020-0075347 | A | 26 June 2020 | (Family: none) | | | |

Form PCT/ISA/210 (patent family annex) (January 2015)

**REFERENCES CITED IN THE DESCRIPTION**

**Patent documents cited in the description**

- JP SHO5865009 A
- JP 2015198999 A
- JP 2016193430 A